# EUROPEAN PATENT SPECIFICATION

(11) **EP 1 200 381 B1**
(45) Date of publication and mention of the grant of the patent: **21.01.2004**
(21) Application number: 00950645.2
(22) Date of filing: 26.07.2000
(51) Int. Cl.: C07C 45/64, C07C 47/19

(54) **PROCESS FOR THE PREPARATION OF 3-HYDROXYPROPANAL**
VERFAHREN ZUR HERSTELLUNG VON 3-HYDROXYPROPANAL
PROCEDE RELATIF A L'ELABORATION DE 3-HYDROXYPROPANAL

(30) Priority: 30.07.1999 US 146421 P; 25.08.1999 US 382998
(43) Date of publication of application: 02.05.2002
(73) Proprietor: E.I. DU PONT DE NEMOURS AND COMPANY, Wilmington Delaware 19898 (US)
(72) Inventor: HAAS, Thomas, D-60322 Frankfurt (DE); HAHM, Torsten, D-63456 Hanau-Klein-Auheim (DE); VANHEERTUM, Rudolf, D-63796 Kahl (DE); HOFEN, Willi, D-63517 Rodenbach (DE); DEUSSER, Liane, D-64390 Erzhausen (DE)
(74) Representative: Carpmaels & Ransford
(86) International application number: PCT/US2000/020192
(87) International publication number: WO 2001/009073

(56) References cited:
- DE-C- 922 166
- US-A- 5 171 898
- PATENT ABSTRACTS OF JAPAN vol. 1996, no. 10, 31 October 1996 (1996-10-31) & JP 08 143502 A (NIPPON SHOKUBAI CO LTD), 4 June 1996 (1996-06-04) cited in the application

## Description

### FIELD OF THE INVENTION

The invention concerns a process for the preparation of 3-hydroxypropanal.

### TECHNICAL BACKGROUND

3-Hydroxypropanal is an intermediate product in the preparation of 1,3-propane diol. 1,3-Propane diol may be further processed to form polyesters, for example poly(trimethylene terephthalate), which is useful for the spinning of fibers, or may be processed to other articles of commerce such as polyols.

It is known that 3-hydroxypropionaldehyde (3-hydroxypropanal) can be prepared by hydration of acrolein with water in the presence of a chelate-forming ion exchanger at temperatures of 30 to 120°C and pressures of 1 to 20 bars as described in U.S. Patent No. 5,171,898, herein incorporated by reference.

U.S. Patent No. 5,284,979 discloses the hydration of 2-alkenals to 3-hydroxyalkanals in a homogeneous phase in the presence of an acid catalyst and a dissolved acid-base buffer which results in a pH of from 2 to 5. A preferred acid base buffer is propanoic acid/triethyl ammonium propionate.

EP 0713853 discloses the addition of oxalic acid to a process for the hydration of acrolein to 3-hydroxypropanal in the presence of a lead-containing ion exchange resin.

Japanese Patent application Kokai No. H 8-143502 discloses the preparation of 3-hydroxyalkanals by the hydration of unsaturated aldehydes in the presence of a metal-carrying ion-exchange resin with the addition of a carboxylic acid to the reaction mixture. Mono and polycarboxylic acids are broadly disclosed, a dicarboxylic acid such as oxalic acid is especially preferred.

DE 922 166 discloses a process for converting olefinic aldehyde into oxyaldehydes using an acid hydratation catalyst.

The known processes have the disadvantage that the catalysts used do not exhibit life times as long as desired. Life time is impaired because of depositions of acrolein polymers on the ion exchanger bed occurring over the course of operation. The acrolein polymers create an increase in pressure differential across the ion exchanger bed and hence a drop in conversion of the unsaturated aldehyde. The ion exchanger must, therefore, be renewed periodically after undesirably short periods of time.

Achievement of economic continuous operation, however, requires an adequately long life time. An object of this invention, therefore, is to improve the known process for the preparation of 3-hydroxypropanal by providing adequately long catalyst life times.

### SUMMARY OF THE INVENTION

The present invention is a process for the preparation of 3-hydroxypropanal by hydration of acrolein in the presence of an ion exchange resin, wherein acrolein and water are reacted using a chelate-forming ion exchanger which contains, bound to the polymer matrix of the polymeric resin, anchor groups of the general formula 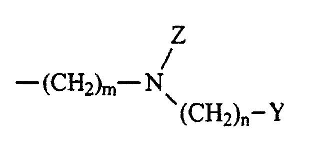 wherein
Z is H, C₁-C₆ alkyl, -CH₂-CH(CH₃)-Y' or -(CH₂)ₒ-Y',
Y and Y' are equal or different: -COOH, -OH, pyridyl, or P(O)(OH)₂, wherein the acidic functional groups may be present, in part, in the form of their salts with alkali, alkali earth or earth metals,
m is 0, 1, 2 or 3,
n is 1, 2 or 3 for Y = -COOH, pyridyl or -P(O)(OH)₂; 2 or 3 for Y = OH, o is 1, 2 or 3 for Y' = COOH, pyridyl or -P(O)(OH)₂; 0, 2 or 3 for Y' = -OH,
wherein said process is carried out with carboxylic acid in the reaction mixture. The acrolein and water are reacted in a weight ratio of 1:2 to 1:20, at 30 to 120°C and at a pressure in the range of 1 to 20 bar.

The amount of carboxylic acid is from 1 ppm to 50,000 ppm, more preferably 10 ppm to 5,000 ppm, by weight in the reaction mixture, and is sufficient to yield a pH of from 1 to 5.5, more preferably from 4 to 5.

The carboxylic acid is an aliphatic carboxylic acid containing from 3 to 8 carbon atoms, and preferably propanoic acid or propenoic acid.

The process may further comprise removing carboxylic acid from the hydrated reaction mixture as an azeotrope with water. After start-up, some or all of the carboxylic acid may be added to the reaction mixture from a recycle stream.

The process of the invention has the advantage that the addition of the carboxylic acid prevents an increase in the pressure differential in the reactor. Drop-off in conversion in the reactor is reduced significantly. Both these effects produce a distinctly longer life time for the catalyst.

### DETAILED DESCRIPTION OF THE INVENTION

Herein, the transitional phrases "comprising", "consisting essentially of" and "consisting of" define the scope of the invention with respect to what unrecited additional components or steps, if any, are included within the scope of the claim. The transitional term "comprising", which is synonymous with "including," "containing," or "characterized by," is inclusive or open - ended and does not exclude additional, unrecited elements or method steps. The transitional phrase "consists essentially of" limits the scope of a claim to the specified materials or steps "and those that do not materially affect the basic and novel characteristic(s)" of the claimed invention. The transitional phrase "consisting of" excludes any element, step, or ingredient not specified in the claim. When the phrase "consists essential of" or "consists of" appears in a clause of the body of a claim, rather than immediately following the preamble, it limits only the element set forth in that clause; other elements are not excluded from the claim as a whole.

As used herein, the term "earth metal" is intended to designate the elements Al, Sc, Y, La and the 14 lanthanides; see Roempps Chemie-Lexikon.

By "acrolein" applicants are referring to any acrolein useful in the invention. Acrolein is available from Degussa-Hüls AG, Frankfurt, Germany, Aldrich Chemical Co., Milwaukee, Wisconsin ("Aldrich") and Fluka Chemical Corp., Milwaukee, Wisconsin.

It is well known that polymerization inhibitors are frequently present in the acrolein, or may be added to the acrolein-water mixture. The polymerization inhibitors are generally added to the acrolein so that it will not polymerize during storage, shipping or transportation. Examples include hydroquinone, hydroquinone monomethylether or butylated phenols. Polymerization inhibitor, such as hydroquinone, is preferably present in acrolein in amount of about 200 - about 1000ppm, and most preferably about 500 ppm. Acrolein may also contain small amounts of acetic aldehyde and other impurities.

Chelate-forming ion exchangers useful in this invention are described in U.S. Patent No. 5,171,898, which is incorporated herein in its entirety by reference.

The polymer matrix of the polymer resin is preferably based on a copolymerizate of styrene and divinyl benzene but can also be an acrylic polymer or copolymer of acrylic compounds and allyl compounds as well as be a polymer of a functionalized epoxide. The anchor groups can be introduced into the polymerization resins in a known manner into the polymer matrix, e.g. (a) by means of chloromethylation of the polymer with subsequent reaction with e.g. glycine, sarcosine, imino diacetic acid or imino dipropionic acid, ethanol amine, diethanol amine or ethanol amine monoacetic acid or (b) by means of nitration, reduction and reaction e.g. with chloroacetic acid. In individual instances a polymerizable monomer containing the anchor group can be polymerized in common with other monomers capable of copolymerization--note for example the copolymerization of N-allylimino dipropionic acid with acrylonitrile. Ion exchangers in which Y and/or Y' signify the group --(O)P(OH)CH.₂OH are known from EP-A 352,949 (GB 017051 of Aug. 18, 1988). Ion exchangers containing the picoyl amine groups are known from U.S. Pat. No. 4,031,038.

Macroporous polymerization resins based on styrene/divinyl benzene copolymers are used with preference; the amino group is preferably bound via a methylene group --m is thus 1 here-- to the aromatic matrix. Especially suitable chelate-forming ion exchangers exhibit as anchor group the methylene imino diacetic acid group with n, m, o=1 and Z= CH.₂COOH and Y= -COOH and it is known that a certain amount of the anchor groups can consist of amino acetic acid functions, that is, m, n=1, Z=H and Y = -COOH.

The chelate-forming ion exchangers with at least one acid group in the anchor group can be used in the form of the free acid (H form); a part of the acid groups --especially up to one third of the total exchange capacity of the resin-- can also be present in the form of an alkali-, alkaline-earth- or earth-metal salt. Ion exchangers whose acid groups are present essentially in the H form are especially preferred.

The conversion of an exchanger supplied in the Na form into the H form takes place in the manner customary for ion exchangers. The adjustment of a certain charge state with metal cations can take place from the H form by means of the addition of the appropriate amount of metal hydroxide, dissolved or suspended, in a suspension of the resin in water. However, an exchanger partially present in the H- and Na form can be obtained by means of the addition of an amount of acid to the Na form of the exchange resin which amount is not sufficient for a complete exchange of the Na ions. In both instances the ion exchange resin is freed of soluble salts and other soluble components before its use by means of washing with deionized water. The exchange capacity can be within a broad range. However, exchangers with a capacity in a range of approximately 1 to 3 equivalents (H form) per liter exchange resin have proven themselves to be especially suitable. The capacity is a measure for the density of the chelate-forming anchor groups in the exchange resin.

Among the ion exchangers in which Y and/or Y' signifies/signify the pyridyl group, those with anchor groups with the formula: 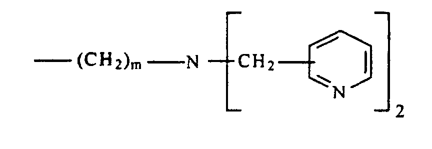 or 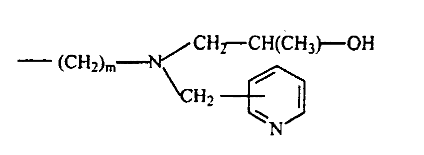 in which m signifies 0, 1, 2 or 3, especially 1, are preferred.

Preferred carboxylic acids are aliphatic carboxylic acids containing from 2 to 8 carbon atoms. Especially preferred carboxylic acids are propenoic acid and propanoic acid. Most preferred is propanoic acid. Propanoic acid is available from Aldrich.

The person of ordinary skill in the art will readily recognize that by "carboxylic acid" reference is being made to carboxylic acid itself, and not a two component acid-base buffer or other such additive. Other additives may be used with the carboxylic acid of this invention. The person of ordinary skill in the art will readily recognize that while a base could be added to control pH or to provide other benefits, bases will materially affect the basic and novel characteristics of the invention since they often promote the polymerization of acrolein and thus would clog the ion exchanger and increase its pressure differential.

The invention may be carried out using the processes described in U.S. Patent No. 5,171,898.

In carrying out the invention acrolein and water are supplied in a weight ratio of 1:2 to 1:20, especially 1:3 to 1:10 and preferably 1:3 to 1:6 to the hydration stage. The conversion to 3-hydroxypropionaldehyde takes place in a temperature range of 30°C to 120°C. A temperature in a range of 30 to 90°C is preferred; a temperature below 30°C generally results in longer reaction times whereas a temperature above 90°C results in a reduced selectivity and problems regarding the service life of the exchange resins. It is especially preferred if the hydration takes place at 30°C to 80°C.

The amount of the carboxylic acid in the hydration reaction mixture is from 1 ppm to 50,000 ppm, preferably 10 ppm to 5,000 ppm. An amount of carboxylic acid is used to maintain the pH of the reaction mixture in the range of from 1 to 5.5, preferably in the range of from 4 to 5.

Carboxylic acid will normally need to be added when the process is started and may need to be added later on. However, after the first addition it may not be necessary to add more carboxylic acid. The reason is believed to be that residual carboxylic acid in recycled water and/or small amounts of carboxylic acid (i.e., acrylic (propenoic) acid) in acrolein are sufficient to maintain the desired levels of carboxylic acid. Carboxylic acids which form water azeotropes are especially useful since these carboxylic acids recycle nearly completely.

In the temperature range below the boiling point of acrolein, the reaction can take place at normal pressure or at moderate pressure. In the case of reaction temperatures around or above the boiling point of acrolein, the work is performed under a pressure in a range of 1 to 20 bars. In the preferred temperature range of 30 to 90°C, a pressure in a range of 1 to 5 bars is preferred.

The hydration is generally carried out up to an acrolein conversion in a range of 30 to 90% or above; a conversion of 40 to 90% and especially 50 to 80% is preferred.

The hydration can take place either discontinuously or continuously and known reactors such as agitator reactors, loop reactors, floating bed reactors, fluid bed reactors and fixed bed reactors can be used. The last-named reactors are preferred over loop reactors and agitator reactors. The residence time and temperature in a fixed bed reactor containing a chelate-forming ion exchanger are controlled in such a manner that the desired acrolein conversion is achieved with a single passage of the reaction mixture through the reactor.

After separation of the ion exchanger, which usually takes place by means of sedimentation or filtration or results by itself when using a resin bed (as is customary, for example, in softened water preparation), the reaction mixture is freed, to the extent necessary, of non-reacted acrolein. The separation of the acrolein can be realized in a known manner, especially by means of distillation, preferably under reduced pressure and temperatures below 80°C. The recovered acrolein can be fed back into the process after stabilization. The practically acrolein-free hydroxypropionaldehyde solution obtained can be reconcentrated before hydrogenation e.g., via a thin-layer evaporator.

A special advantage of the use of propanoic acid as the carboxylic acid in the process of the present invention is the fact that carboxylic acid (e.g., propanoic acid) can be removed from the hydrated reaction mixture as an azeotrope with water and thus is not carried downstream to the subsequent (hydrogenation) stage.

In one preferred process, the feed is filtered before the catalyst bed to remove particulates/polymers (especially polymers dissolved in pure acrolein) using known techniques. Static mixers may be used to ensure adequate mixing of acrolein and water.

The process of the invention has the advantage that the addition of carboxylic acid prevents an increase in the pressure differential in the reactor. Drop-off in conversion in the reactor is reduced significantly. Both these effects produce a distinctly longer life time for the catalyst. It has been observed that there is less polymerization of acrolein when the carboxylic acid is added according to the invention, and thus it is believed that the carboxylic acid is preventing or reducing the polymerization of acrolein with itself and thus reducing clogging of the reactor/ion exchanger.

Experiments to demonstrate the reaction of acrolein to 3-hydroxypropanal are performed in a tubular apparatus under continuous flow. The reactor consists of a double-jacketed glass tube of 3 m length and 76 mm inner diameter. The reactor is charged with ion exchanger Lewatit TP 208 (H form) (Bayer AG, Germany). The aqueous solution is preheated to reaction temperature and pumped through the catalyst bed from the bottom up. The reactor is maintained at temperature by a thermostat. A pressure of 2.5 bar absolute is set on the exit of the reactor. Both feed and product solutions are analyzed by gas chromatography. Analyses are then used to determine conversion and selectivity of the reaction. The product solution is then hydrogenated according to the procedure of U.S. Patent No. 5,334,778 and the hydrogenation product is distilled. After completing distillation, the content of propanoic acid in the H₂O-distillate and the purity of 1,3-propanediol in the 1,3-propanediol distillate were determined by gas chromatography.

All percentages are by weight unless otherwise indicated.

### COMPARATIVE EXAMPLE 1

The reactor tube was charged with 10.5 I ion exchanger. An aqueous acrolein solution (~ 500 ppm hydroquinone) at a concentration of 17.5 wt. % was pumped through the ion exchanger (Lewatit TP 208, H form, Bayer AG, Germany), at a volume flow of 6.5 l/hour. The average reactor temperature was 69°C. The pH-value of the solution was 5.8 prior to exposure to the ion exchanger. After an experimental duration of about 10 hours, the measured pressure differential across the reactor was 0.4 bar. Conversion was 54.5 % and selectivity was 81.8%. After an additional interval of 144 hours, the pressure differential was 0.8 bar, conversion was 49.3% and selectivity 81.6%. No propanoic acid was detected in the H₂O-distillate. Purity of the 1,3-propane diol distillate was 99.7 GC-area %.

### EXAMPLE 1

The reactor tube was charged with 10.5 1 catalyst. Acrolein (∼500 ppm hydroquinone) concentration in the aqueous solution was 17.5% and 100 ppm of propanoic acid was added. The solution was pumped through the catalyst bed (Lewatit TP 208, H form ) at a rate of 6.5 l/hour. Average reactor temperature was 69°C and the pH value ahead of the reactor was 4.1. After an experimental duration of about 10 hours, pressure differential on the reactor of 0.4 bar was reached. Conversion was 54.4% ,and the selectivity with respect to 3-hydroxypropanal was 81.9%. After an additional interval of 316 hours, the pressure differential was measured at 0.4 bar, conversion was 53.9 % and selectivity with respect to 3-hydroxypropanal was 81.4 %. Eighty percent of the propanoic acid charged was found in the H₂O-distillate. Purity of the 1,3-propane diol distillate was 99.8 GC-area %.

When Comparison Example 1 and Example 1 are compared, it is evident that the addition of propanoic acid prevents an increase in reactor pressure differential and also significantly reduces the drop in conversion in the reactor with extended reaction times. Both lead to significantly prolonged catalyst life time.

## Claims

1. A process for the preparation of 3-hydroxypropanal by hydration of acrolein in the presence of an ion exchanger, comprising reacting acrolein and water in weight ratio of 1:2 to 1:20, at 30°C to 120°C and at a pressure in the range of 1 to 20 bar using a chelate-forming ion exchanger which contains, bound to a polymer matrix of a polymeric resin, anchor groups of the general formula 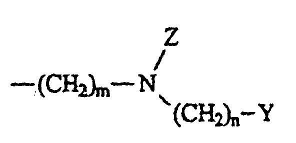 wherein
Z is H, C₁-C₆ alkyl, -(CH₂-CH(CH₃)-Y' or -(CH₂)ₒ-Y',
Y and Y', which are the same or different, are -COOH, -OH, pyridyl or - P(O)(OH)₂, wherein the acidic functional groups may be present, in part, in the form of their salts with alkali, alkali earth or earth metals,
m is 0, 1, 2 or 3,
n is 1, 2 or 3 for Y = -COOH, pyridyl or -P(O)(OH)₂; 2 or 3 for Y = -OH,
o is 1, 2 or 3 for Y' - COOH, pyridyl or -P(O)(OH)₂; 0, 2 or 3 for Y' = -OH,
further comprising carrying out the process with from 1 ppm to 50,000 ppm by weight of an aliphatic carboxylic acid containing from 3 to 8 carbon atoms and wherein the reaction mixture is at a pH of from 1 to 5.5.

2. The process of Claim 1 wherein the amount of carboxylic acid in the reactant mixture is from 10 ppm to 5,000 ppm by weight.

3. The process of Claim 1 or 2 wherein the amount of carboxylic acid is sufficient to yield a pH of from 4 to 5.

4. The process of any of Claims 1-3 wherein the carboxylic acid is propanoic acid.

5. The process of any of Claims 1-3 wherein the carboxylic acid is propenoic acid.

6. The process of any of the preceding claims wherein the process comprises carrying out the process using an additive consisting essentially of the carboxylic acid.

7. The process of claim 6 wherein the process consists essentially of adding the additive to the reaction mixture.

8. The process of any of the preceding claims further comprising removing carboxylic acid from the hydrated reaction mixture as an azeotrope with water.

9. The process of any of the preceding claims wherein after start-up some or all of the carboxylic acid is added to the reaction mixture from a recycle stream.

## Patentansprüche

1. Verfahren zur Herstellung von 3-Hydroxypropanal durch Hydratation von Acrolein in Anwesenheit eines Ionenaustauschers, umfassend Umsetzen von Acrolein und Wasser im Gewichtsverhältnis von 1:2 bis 1:20 bei 30°C bis 120°C und bei einem Druck in dem Bereich von 1 bis 20 bar unter Verwendung eines chelatbildenden Ionenaustauschers, welcher, gebunden an eine Polymermatrix eines polymeren Harzes, Ankergruppen der allgemeinen Formel 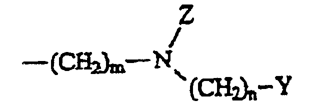 enthält, wobei
Z H, C₁-C₆-Alkyl, -CH₂-CH(CH₃)-Y' oder -(CH₂)ₒ-Y' ist,
Y und Y', welche gleich oder verschieden sind, -COOH, -OH, Pyridyl oder -P(O)(OH)₂ sind, wobei die sauren funktionellen Gruppen teilweise in Form ihrer Salze mit Alkali-, Erdalkali- oder Erdmetallen vorhanden sein können,
m 0, 1, 2 oder 3 ist,
n 1, 2 oder 3 für Y = -COOH, Pyridyl oder -P(O)(OH)₂; 2 oder 3 für Y = -OH ist,
o 1, 2 oder 3 für Y' = -COOH, Pyridyl oder -P(O)(OH)₂; 0, 2 oder 3 für Y' = -OH ist,
weiterhin umfassend Ausführen des Verfahrens mit von 1 ppm bis 50000 ppm, bezogen auf das Gewicht, einer aliphatischen Carbonsäure, enthaltend von 3 bis 8 Kohlenstoffatome, und wobei das Reaktionsgemisch bei einem pH von 1 bis 5,5 ist.

2. Verfahren nach Anspruch 1, wobei der Anteil von Carbonsäure in dem Reaktantengemisch von 10 ppm bis 5000 ppm, bezogen auf das Gewicht, beträgt.

3. Verfahren nach Anspruch 1 oder 2, wobei der Anteil von Carbonsäure ausreichend ist, um einen pH von 4 bis 5 zu ergeben.

4. Verfahren nach einem der Ansprüche 1-3, wobei die Carbonsäure Propansäure ist.

5. Verfahren nach einem der Ansprüche 1-3, wobei die Carbonsäure Propensäure ist.

6. Verfahren nach einem der vorhergehenden Ansprüche, wobei das Verfahren Ausführen des Verfahrens unter Verwendung eines Zusatzstoffes, bestehend im wesentlichen aus der Carbonsäure, umfaßt.

7. Verfahren nach Anspruch 6, wobei das Verfahren im wesentlichen aus dem Hinzufügen des Zusatzstoffes zu dem Reaktionsgemisch besteht.

8. Verfahren nach einem der vorhergehenden Ansprüche, weiterhin umfassend Entfernen der Carbonsäure aus dem hydratisierten Reaktionsgemisch als Azeotrop mit Wasser.

9. Verfahren nach einem der vorhergehenden Ansprüche, wobei nach dem Beginn etwas oder alles von der Carbonsäure aus einem Rücklaufstrom zu dem Reaktionsgemisch hinzugefügt wird.

## Revendications

1. Procédé pour la préparation de 3-hydroxypropanal par hydratation d'acroléine en présence d'un échangeur d'ions, comprenant la réaction d'acroléine et d'eau dans le rapport pondéral de 1:2 à 1:20, à une température de 30°C à 120°C et à une pression dans la plage de 1 à 20 bars, en utilisant un échangeur d'ions formateur de chélate qui contient, liés à une matrice polymère d'une résine polymère, des groupes d'ancrage de formule générale : 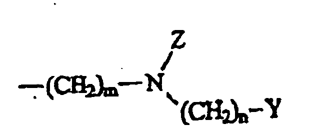 dans laquelle :
Z est H, un alkyle en C₁-C₆, -CH₂-CH(CH₃)-Y' ou - (CH₂)ₒY',
Y et Y', qui sont identiques ou différents, sont -COOH, -OH, pyridyle ou -P(O)(OH)₂, dans lesquels les groupes fonctionnels acides peuvent être présents, en partie, dans la forme de leurs sels avec des métaux alcalins, alcalinoterreux ou terreux,
m est 0, 1, 2 ou 3,
n est 1, 2 ou 3 pour Y=-COOH, pyridyle ou -P(O)(OH)₂; 2 ou 3 pour Y=-OH,
o est 1, 2 ou 3 pour Y'=-COOH, pyridyle ou -P(O)(OH)₂; 2 ou 3 pour Y'=-OH,
comprenant par ailleurs la réalisation du procédé à partir de 1 ppm à 50 000 ppm en poids d'un acide carboxylique aliphatique contenant 3 à 8 atomes de carbone, et dans lequel le mélange réactionnel se fait à pH de 1 à 5,5.

2. Procédé selon la revendication 1, dans lequel la quantité d'acide carboxylique dans le mélange réactionnel est de 10 ppm à 5000 ppm en poids.

3. Procédé selon la revendication 1 ou 2, dans lequel la quantité d'acide carboxylique est suffisante pour donner un pH de 4 à 5.

4. Procédé selon l'une quelconque des revendications 1 à 3, dans lequel l'acide carboxylique est l'acide propanoïque.

5. Procédé selon l'une quelconque des revendications 1 à 3, dans lequel l'acide carboxylique est l'acide propénoïque.

6. Procédé selon l'une quelconque des revendications précédentes, dans lequel le procédé comprend la réalisation du procédé en utilisant un additif constitué essentiellement de l'acide carboxylique.

7. Procédé selon la revendication 6, dans lequel le procédé consiste essentiellement à ajouter l'additif au mélange réactionnel.

8. Procédé selon l'une quelconque des revendications précédentes, comprenant en outre l'élimination de l'acide carboxylique du mélange réactionnel hydraté sous la forme d'un azéotrope avec de l'eau.

9. Procédé selon l'une quelconque des revendications précédentes, dans lequel, après démarrage, une partie ou la totalité de l'acide carboxylique est ajoutée au mélange réactionnel à partir d'un courant de recyclage.
